(19) Europäisches Patentamt
European Patent Office
Office européen des brevets

(11) **EP 4 209 596 A1**

(12) **EUROPEAN PATENT APPLICATION**

(43) Date of publication:
**12.07.2023 Bulletin 2023/28**

(21) Application number: **22150566.2**

(22) Date of filing: **07.01.2022**

(51) International Patent Classification (IPC):
**C12Q 1/6825** (2018.01)

(52) Cooperative Patent Classification (CPC):
(C-Sets available)
**C12Q 1/6825** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Bioanalizes sistemos, UAB**
 **10224 Vilnius (LT)**
• **Laboratorija 1, UAB**
 **01114 Vilnius (LT)**

(72) Inventors:
• **Ratautas, Dalius**
 **Vilnius (LT)**
• **Serapinas, Skomantas**
 **Vilnius (LT)**

• **Gineityte, Justina**
 **Vilnius (LT)**
• **Butkevicius, Marius**
 **Vilnius (LT)**
• **Danilevicius, Rapolas**
 **Vilnius (LT)**
• **Dagys, Marius**
 **Vilnius (LT)**

(74) Representative: **Petniunaite, Jurga**
 **AAA Law**
 **P.O. Box 33**
 **A. Gostauto street 40B**
 **03163 Vilnius (LT)**

Remarks:
The complete document including Reference
Table(s) and the Sequence Listing(s) can be
downloaded from the EPO website

(54) **ELECTROCHEMICAL SYSTEM FOR THE RAPID AND SENSITIVE DETECTION AND QUANTIFICATION OF NUCLEASE-LIKE ACTIVITY EXHIBITING ENZYMES**

(57) The presented invention provides a rapid and sensitive system for the detection and quantification of nuclease-like activity enzymes. Invention involves a nucleic acid sequence composed of both single-stranded and double-stranded nucleic acid parts with the covalently immobilized redox-active label (an oligonucleotide target) which can be cleaved by a nuclease-like activity enzyme(s); a specific surface design electrode with the immobilized oligonucleotide probe; and a specific electrochemical measurement and signal analysis method. The hybridization of the oligonucleotide target on the electrode is observed by measuring the surface concentration and/or amount of the redox-active label and is directly related to the concentration of enzyme(s) demonstrating a nuclease-like activity.

Fig. 3

EP 4 209 596 A1

(52) Cooperative Patent Classification (CPC): (Cont.)

C-Sets
**C12Q 1/6825, C12Q 2521/301, C12Q 2563/113,
C12Q 2563/113, C12Q 2565/607**

**Description**

**Technical Field**

This application relates to the field of enzyme detection, more specifically - nuclease-like activity detection devices.

**Background Art**

Nucleases are enzymes which belong to the hydrolase class and cleave (hydrolyse) the phosphodiester bonds of nucleic acids (DNA and/or RNA), thus resulting in bond breaks (or chain shortenings) of ss/dsDNA and/or ss/dsRNA molecules. Additionally, nucleases are also classified as endonucleases (cleave the phosphodiester bond within the nucleic acid chain) and exonucleases (cleave the phosphodiester bond from the end of nucleic acid chain). Both types of nucleases are common in the environment, since they are produced by living cells (i.e., bacteria, animals, plants, fungus, etc.), are commonly found in the bodily fluids (e.g., saliva, blood) and are relatively stable outside the cell in the environment. The detection and quantification of nucleases is an important challenge for medical and the biotechnological fields. For medical applications, recent research papers reported that nuclease activity in various bodily fluids is related with the diseases such as cancers (Kruspe et al., 2017) or bacterial infections (Flenker et al., 2017). In the field of biotechnology nucleases cause problems related to contamination, product, and medicine quality control. Nuclease control is especially important in the fields of molecular biology and next generation vaccines, based on recombinant RNA technology, development, and production. In those cases, the presence of nucleases could significantly influence the quality of the product and/or make the results of scientific experiments irregular, irreproducible and/or unreliable. For example, it was reported that nuclease contamination was very common in molecular biology systems typically used for oxygen-scavenging purposes together with DNA and/or RNA, and it could have significantly influenced the received results (Senavirathne et al., 2015). In addition, major biotechnological companies test for nuclease contamination in their products routinely.

To this regard, various methods were developed for the detection and quantification of nuclease and/or nuclease-like activity in solutions. The most common methods used for nuclease and/or nuclease-like activity detection are based on fluorescence and radiolabelling. Fluorescence-based methods usually rely on fluorescence quenching and are implemented by using a specific sequence oligonucleotide probe modified by a fluorophore at one end and a quencher at the other. The fluorophore fluorescence is significantly decreased where the quencher is present. The designed probe is placed together with the solution of analysis (solution where nuclease-like activity is investigated) and kept for a specific amount of time (usually 1-18 hours). If a solution contains no nucleases, the fluorescence does not change since the probe remains intact and fluorophore fluorescence is reduced by quencher. In case the nucleases are present, they cleave the probe, and the fluorophore is separated from the quencher - this separation results in the increased fluorescence of the solution. The described method was reported in scientific papers (Zhang et al., 2021)(He et al., 2013) as well as patent applications (EP2751567B1; US7276337B2; US2020/0017898A1; WO2014/207515A1). The fluorescence-based detection methods have a few disadvantages: probes are expensive, since they require fluorophore and quencher-modified nucleic acid, expensive equipment for fluorescence measurements and a lot of manual work. Additionally, radiolabelling methods for nuclease-like activity detection are used. These methods are based on gel electrophoresis of radiolabelled-nucleic acid fragment. Usually, nucleic acid sequence is labelled using 32P and then incubated up to 18 hours with the sample where nuclease-like activity is investigated. In case no nuclease is present, the single peak of radiolabelled nucleic acid should be visible after the electrophoresis. If a sample contained nucleases, they cleaved the nucleic acid in various fragments, thus the position of radiolabel (32P) becomes distorted. Though the 32P method is extremely sensitive (up to 0.1 pg/mL or $10^{-6}$ U/mL) it requires a lot of manual work, advanced laboratory equipment and usage of hazardous radioactive compounds. To make the measurements of nuclease-like activity cheaper, various less labour-intensive electrochemical methods were reported in non-patent literature, however, they are not used for nuclease detection commercially (Sato et al., 2009)(Sato and Takenaka, 2014)(Ding and Qin, 2013). Most of the described methods were based on signal-off method and were implemented using specific electrode with immobilized redox-labelled nucleic acid. The electrode was placed inside a solution, where nucleases were suspected and kept for 0.5-18 hours. In case no nuclease was present electrode potential-current response was unchanged. If nucleases were present, they cleaved the immobilized sequence, thus removing redox-active probe from the electrode surface and, in turn, reducing the electrochemical potential-current signal. The reported methods were attractive since the majority of the work could be automated (less labour intensive), however, typically electrochemical nuclease detection methods resulted in significantly lower detection limits compared to the fluorescent- or radiolabelling-based.

Present technologies for nuclease detection are time-consuming, require a lot of manual work, are expensive and use toxic radioactive chemicals. New alternative technologies are not nearly as developed and lack sensitivity required for relevant applications. As a result, a rapid, inexpensive, non-toxic, sensitive, labour non-intensive measurement method for nuclease-like activity is required.

**Summary of the Invention**

The presented invention, in certain embodiments, provides a system for the detection and quantification of nuclease-like activity exhibiting enzymes as well as all the necessary information for the production of nuclease-like activity detection kits for a rapid, cheap, and labour non-intensive detection and quantification of nuclease-like activity. The invention reported herein is directed to the detection and quantification of total nuclease-like activity in a specific sample, thus focused on non-selective and non-specific recognition of nuclease-like activity exhibiting enzymes. However, the invention can easily be embodied for a selective recognition of specific nucleases.

The presented invention, in certain embodiments, provides those components which allow the detection of the nuclease-like activity:

i) a specific design oligonucleotide target containing at least one cleavage site for nuclease-like activity enzyme(s) and modified with one or more redox-active label at 3' or 5' ends or in the chain of the oligonucleotide;

ii) a specific design electrode surface together with the immobilized oligonucleotide probe capable to hybridize the oligonucleotide target;

iii) a hybridization method and specific signal analysis conditions which allow to estimate the presence of the redox-active label on the electrode surface depending on the reduction and/or oxidation currents, total charge, electrochemical potential, or impedance and thus to detect and quantify the nuclease-like activity enzymes.

The detection of nuclease-like activity can be implemented, but not limited to, using components i-iii in described order. The sample which contains enzymes of nuclease-like activity is being placed together with a specific design oligonucleotide target. In a preferred embodiment, a sample is placed with an oligonucleotide target in a specific buffer solution at a specific temperature to maximize the nuclease-like activity of enzyme(s). The possible compositions of those buffer solutions are disclosed further. The sample and the oligonucleotide target should be kept together for a sufficient time for the cleavage of the oligonucleotide target to occur, in case a sample contains the nuclease-like activity enzymes. After a sufficient amount of time, the sample solution with the oligonucleotide target is placed in a solution together with the specific surface design electrode, containing immobilized oligonucleotide probe. The hybridization event takes place between the immobilized oligonucleotide probe on the electrode surface and the oligonucleotide target present in the solution. In a preferred embodiment, the hybridization event is conducted using specific buffer solution at a specific temperature, to maximize the rate of nucleic acid hybridization. The possible compositions of those buffer solutions are disclosed further. In a most preferred embodiment, the hybridization is conducted utilizing the screen-printed electrodes by placing the solution on the electrode surface or by placing the electrode into a solution. The hybridization event is monitored by utilizing specific measurement algorithm which can produce information about the amount and/or concentration of an oligonucleotide target hybridized on the electrode surface and/or the rate of hybridization of oligonucleotide target. In a preferred embodiment, the amount and/or concentration and/or hybridization rate of oligonucleotide target can be received by observing and measuring the reductive and/or oxidative currents and/or electrochemical potentials and/or charges and/or impedance of a redox-active label present within the oligonucleotide target. The described measurements are conducted using electrochemical workstation or potentiostat or other electrochemical potential-current measuring device. In a most preferred embodiment, the measured reductive and/or oxidative current values and/or electrochemical potentials and/or charge and/or impedance are signal-processed using specific algorithm to produce a value of the exact concentration and/or amount and/or hybridization rate of oligonucleotide target with the redox-active label. In case the sample contains the enzymes exhibiting nuclease-like activity the oligonucleotide target with the redox-active label is cleaved. As a result, the measured and/or the signal-processed reductive and/or oxidative current and/or electrochemical potentials and/or charge and/or impedance values are reduced accordingly. In a preferred embodiment, the signal-processed values of the reductive and/or oxidative currents resulted from oligonucleotide target with the redox-active label are directly related to the nuclease-like activity of the enzymes in a sample.

**Brief description of the drawings**

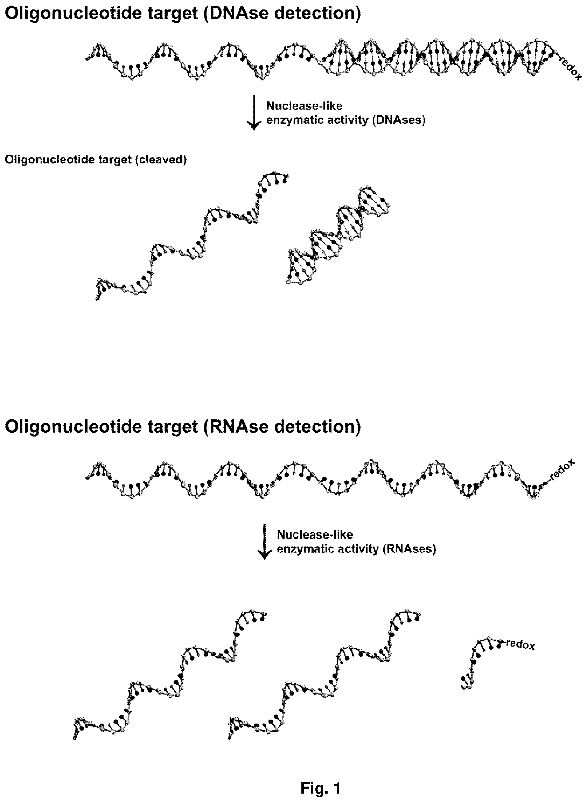

Fig. 1. A drawing of nucleic acid sequence (oligonucleotide target) designed for nuclease-like activity testing. The oligonucleotide target is a DNA and/or RNA oligonucleotide composed of short and long nucleic acid chains. Short chain contains the redox-active label and a region of hybridization (complementarity) to the long chain. The redox-active label is capable for reversible reduction and/or oxidation. The long chain contains a region of hybridization (complementarity) to the short chain and a region of hybridization (complementarity) to the oligonucleotide probe on the electrode surface. When exposed to enzyme(s) which has a nuclease-like activity the oligonucleotide target is cleaved resulting in shorter fragment(s).

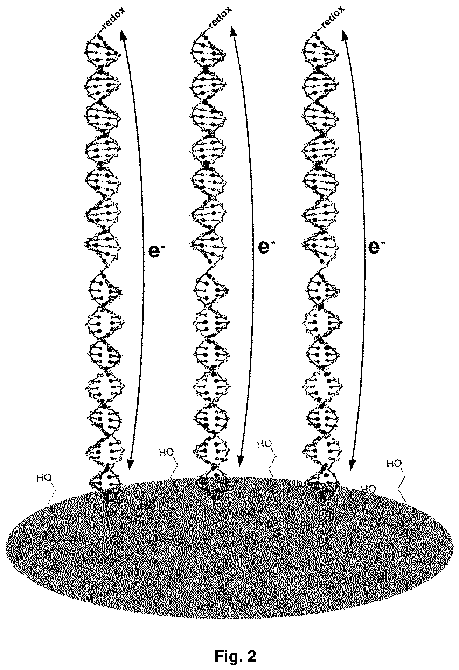

**Fig. 2.** An electrode surface modified with nanomaterials to increase the surface area, the specific oligonucleotide probe and the specific supplemental chemical compounds hybridized with oligonucleotide target unaffected by the nuclease-like activity enzymes. In case oligonucleotide target is unaffected by the nuclease-like activity enzymes, the redox-active label is present at a close proximity to the electrode surface and can be detected by the means of electrochemical analysis.

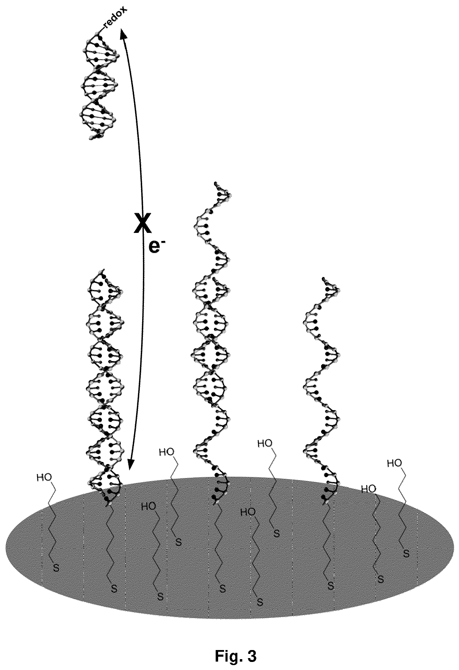

**Fig. 3.** An electrode surface modified with nanomaterials to increase the surface area, the specific oligonucleotide probe and the specific supplemental chemical compounds partially hybridized with oligonucleotide target which is affected by the nuclease-like activity enzymes. In case oligonucleotide target is affected by the nuclease-like activity enzymes, the oligonucleotide target is cleaved, resulting in one or more fragments. The redox-active label is not present at a close proximity to the electrode surface and the signal is decreased.

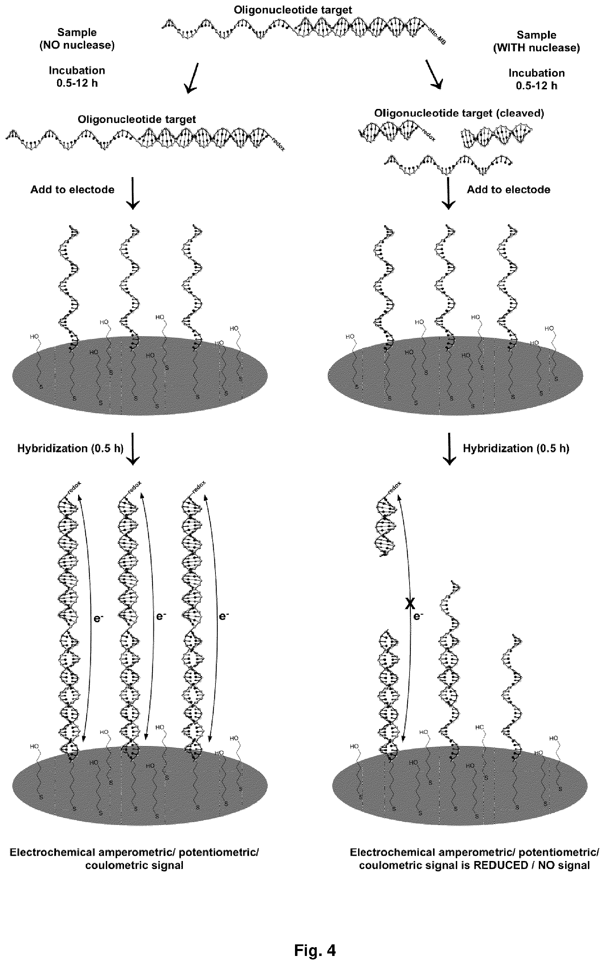

**Fig. 4.** A scheme demonstrating the method for detection and quantification of nuclease-like activity enzyme(s). As a first step the sample is incubated with the oligonucleotide target for 0.5-12 hours. In case no nuclease-like activity is present: the oligonucleotide target remains uncleaved and is hybridized for 0.5 hour with the electrode, which surface is modified with nanomaterials, the specific oligonucleotide probe, and the specific supplemental chemical compounds; the hybridization event is detected and monitored by measuring the signal of redox-active label near the electrode surface. In case nuclease-like activity is present: the oligonucleotide target is cleaved and resulting fragments are hybridized for 0.5 hours with the electrode, which surface is modified with nanomaterials, the specific oligonucleotide probe and the specific supplemental chemical compounds; the hybridization event is detected and monitored by measuring the signal of redox-active label near the electrode surface; the hybridization rate is lower for the cleaved oligonucleotide target due to the redox-active label not being present at close proximity to the electrode surface. The rate of hybridization allows to detect and quantify the nuclease-like activity enzyme(s).

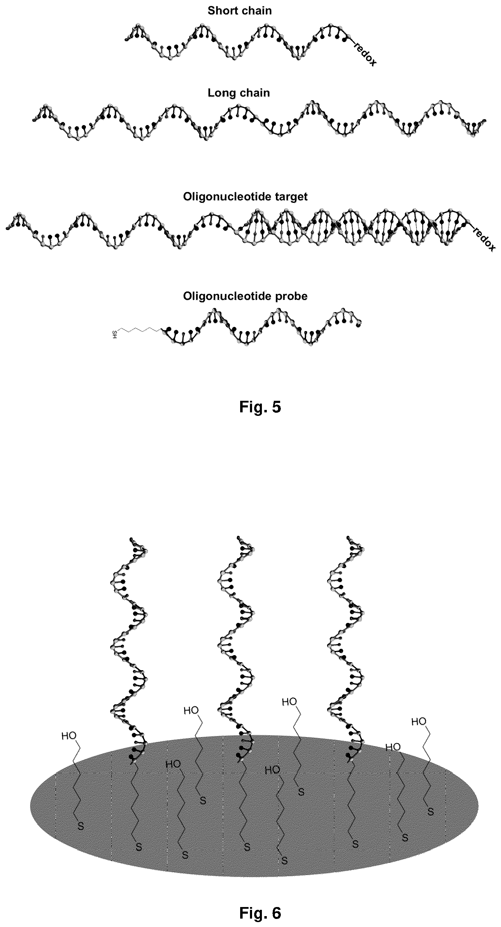

**Fig. 5.** Oligonucleotide structures proposed in this invention. Oligonucleotide target - composed of short chain and the long chain; oligonucleotide probe.

**Fig. 6.** An electrode surface modified with specific nanomaterials (to increase the surface area), the specific oligonucleotide probe (to hybridize with the oligonucleotide target) and the specific supplemental chemical compounds (to protect the surface from the non-selective and non-specific adsorption).

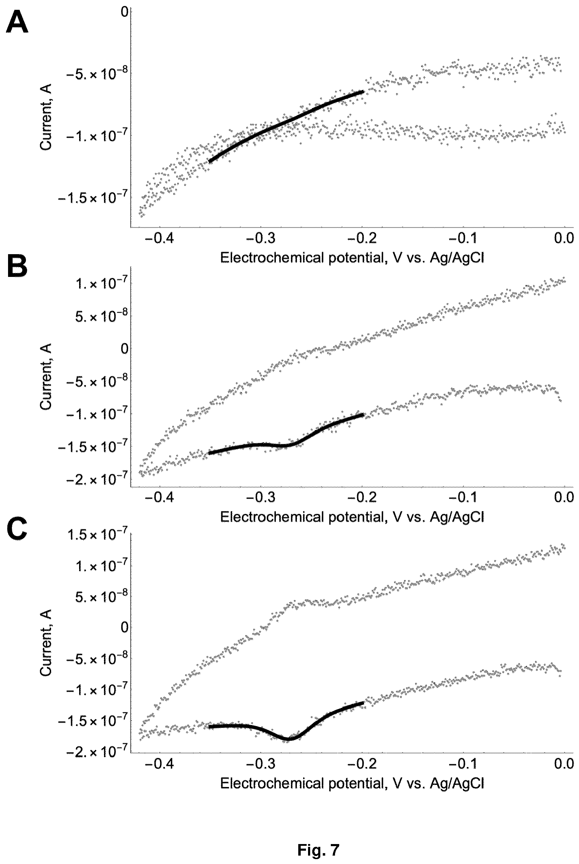

**Fig. 7.** The potential-current curves demonstrating the reductive and oxidative current of the redox-active label on the electrode surface after 0 min (A), 15 min (B) and 30 min (C). The oxidative and reductive current module values increase depending on the hybridization time. The absolute amount of the redox-active label is calculated by taking the specific range of potential-current values from the data points and fitting to an analytical potential-current curve. Black lines in A, B and C parts demonstrate the fitted curves placed on the measured data points. The fitted curves allow to calculate the amount and/or concentration of the redox-active label.

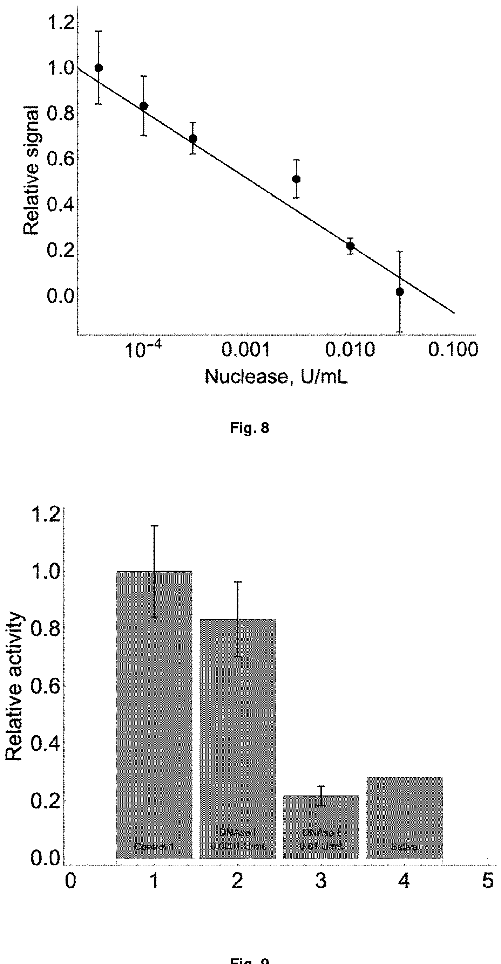

**Fig. 8.** A curve demonstrating a relative signal of the electrode depending on the activity on nuclease-like enzymes. The curve demonstrates a linear dependence based on native logarithm of the nuclease-like activity.

**Fig. 9.** A comparison of the relative electrode signal of various samples, including human saliva sample: (1) control (no nuclease-like activity enzymes), (2) DNAse I 0.0001 U/mL, (3) DNAse I 0.01 U/mL, (4) saliva.

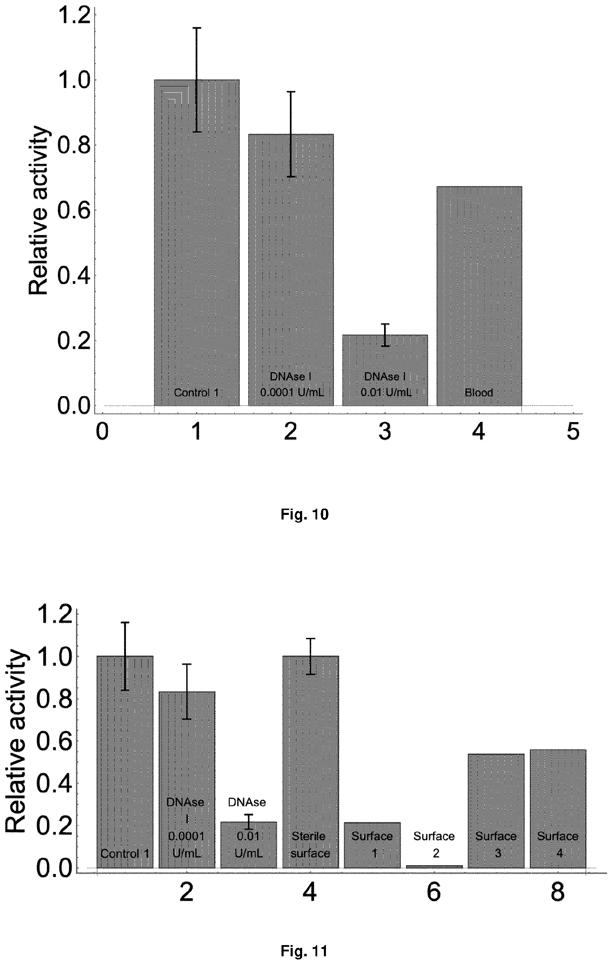

**Fig. 10.** A comparison of the relative electrode signal of various samples, including human blood sample: (1) control (no nuclease-like activity enzymes), (2) DNAse I 0.0001 U/mL, (3) DNAse I 0.01 U/mL, (4) blood.

**Fig. 11.** A comparison of the relative electrode signal of various samples, including samples taken from various surfaces: (1) control (no nuclease-like activity enzymes), (2) DNAse I 0.0001 U/mL, (3) DNAse I 0.01 U/mL, (4) sterile surface (nuclease-like activity was removed due to isopropyl alcohol treatment), (5) surface 1 (touchscreen of a printer), (6) surface 2 (a table of a resting room), (7) surface 3 (a keyboard of a computer), (8) surface 4 (a keyboard of another computer).

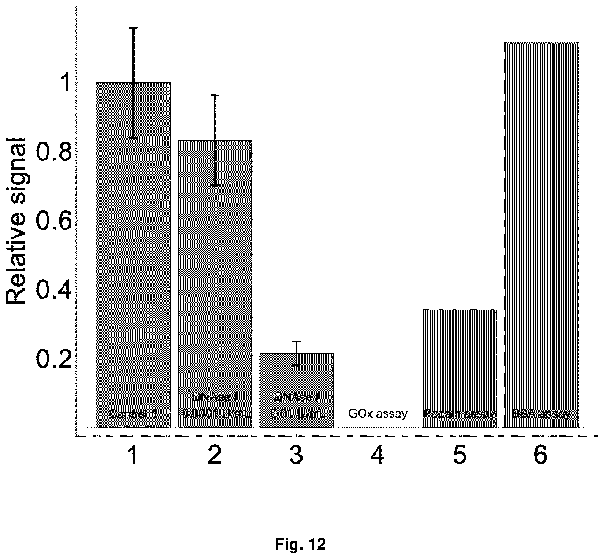

**Figure 12.** A comparison of the relative electrode signal of various samples, including samples taken of commercial protein matrixes: (1) control (no nuclease-like activity enzymes), (2) DNAse I 0.0001 U/mL, (3) DNAse I 0.01 U/mL, (4) sample of glucose oxidase protein matrix, (5) sample of papain protein matrix, (6) sample of bovine serum albumin protein matrix.

## Detailed description of the Invention

The presented invention, in certain embodiments, provides a system for the detection and quantification of nuclease-like activity exhibiting enzymes.

The sample which contains enzymes of nuclease-like activity is being placed together with a specific design oligonucleotide target, only a small amount of sample (0.005-0.5 mL) is needed to analyse the nuclease-like activity. In a preferred embodiment, a sample is placed with a target oligonucleotide in a specific buffer solution at a specific temperature to maximize the nuclease-like activity of specific enzyme or mix of enzymes exhibiting the nuclease-like activity. The sample and the oligonucleotide target are placed in a low-binding tube to minimize nucleic acid or enzyme adsorption to the tube surface. The specific buffer solution is a water-based solution containing low salt concentration up to 0.05 mol/L. The pH of the buffer solution is in range 7.0-8.0 kept by using TRIS, TBE or TAE salts. The two-valence ions such as Be, Mg, Ca, Sr, Mn are added at low concentrations up to 0.02 mol/L to a buffer solution. In a preferred embodiment, the temperature of the solution is kept at 30-40 °C to increase the rate of nuclease cleavage of oligonucleotide target. The sample and the oligonucleotide target should be kept together for a sufficient time for the cleavage of the target nucleotide to occur, in case a sample contains the nuclease-like activity enzymes as demonstrated in **Fig. 1.** In the preferred embodiment, the minimum time required for a cleavage to occur is 0.5 hour. Additional time is given to increase the sensitivity for nuclease-like activity detection and can be increased up to 12 hours. After a sufficient amount of time, the sample solution with the target oligonucleotide is placed to a solution with the specific design electrode containing immobilized oligonucleotide probe. The hybridization event takes place between the immobilized probe oligonucleotide on the electrode and between the target oligonucleotide present in the solution. In a preferred embodiment, the hybridization event is conducted using specific buffer solution at a specific temperature, to maximize the rate of nucleic acid hybridization and the preferred conditions are described below. In a most preferred embodiment, the hybridization is conducted utilizing the screen-printed electrodes by placing the solution on the electrode surface or by placing the electrode into a solution. The hybridization event is monitored by utilizing specific measurement algorithm which can produce information about the amount and/or concentration of an oligonucleotide target hybridized on the electrode surface and/or the rate of oligonucleotide target hybridization. In a preferred embodiment, the amount and/or concentration and/or hybridization rate of oligonucleotide target can be received by observing and measuring the reductive and/or oxidative currents and/or electrochemical potentials and/or charges and/or impedance of a redox-active label present within the oligonucleotide target. The described measurements are conducted using electrochemical workstation or potentiostat or other electrochemical potential-current measuring device. In a most preferred embodiment, the measured reductive and/or oxidative current values and/or electrochemical potentials and/or charge and/or impedance are signal-processed using specific mathematical algorithm to produce a value of the exact concentration and/or amount and/or hybridization rate of oligonucleotide target with the redox-active label. The methods to calculate the exact amount and/or concentration of the redox-active label are described below. In case the sample contains the enzymes exhibiting nuclease-like activity the oligonucleotide target with the redox-active label is cleaved. As a result, the measured and/or the signal-processed reductive and/or oxidative current and/or potentials and/or charge and/or impedance values are reduced accordingly, while the uncleaved oligonucleotide target on the electrode surface does not result in decrease of the above-described parameters. As demonstrated in **Fig. 2** the uncleaved oligonucleotide target hybridizes to the electrode surface and results in significant signal of the redox-active label. In contrast, as demonstrated in **Fig. 3,** the cleaved oligonucleotide target hybridizes slower and/or does not hybridize to the electrode surface and/or hybridizes to the electrode surface without redox-active label and it results in decreased signal of the redox-active label. In a preferred embodiment, the signal-processed values of the reductive and/or oxidative currents resulted from oligonucleotide target with the redox-active label are directly related to the nuclease-like activity of the enzymes in a sample. The signal values can be calibrated using solutions with different nuclease-like activity, thus resulting in a calibration curve. The total design scheme of the preferred embodiment of nuclease-like activity detection and quantification is demonstrated in **Fig. 4.**

**An oligonucleotide target.** A specific design oligonucleotide target is a nucleic acid molecule containing at least one cleavage site for nuclease-like activity enzyme(s) and modified with one or more redox-active label at 3' or 5' ends or in the middle of the chain of the oligonucleotide. A specific design oligonucleotide target is a nucleic acid molecule containing 10-200 nucleotides. In the preferred embodiment for DNase detection, the oligonucleotide target is composed of two chains of deoxyribonucleic acid molecules hybridized together - a short and a long chain (**Fig. 5**). This embodiment maximizes the non-selective and non-specific cleavage of the oligonucleotide targets due to certain deoxyribonuclease-like activity exhibiting enzymes preferring to cleave single-stranded nucleic acids, while others - double stranded nucleic acids. Short chain contains the redox-active label and a region of hybridization (complementarity) to the long chain, the redox-active label is capable of reversible reduction and/or oxidation. In a preferred embodiment, the redox-active label is covalently immobilized to the oligonucleotide phosphate groups and/or nucleoside groups. The redox-active label is a molecule capable of existing within at least two oxidative states, e.g., oxidized, reduced, partly oxidized, partly reduced, etc. Redox-active label can be of inorganic or organic class, a protein, a metal complex, a quantum dot, a graphene-based redox-active species, or any other molecule capable to transfer the charge. In certain embodiments, the redox-active molecule is one of the listed, but not limited, to those compounds: methylene blue, AttoMB2, IRDye®800, IRDye®700, Yakima Yellow®, Texas Red®, Atto Rho13, Cy5.5, Cy5-azide, Azide-fluor 488, Atto655, Atto647N, Atto Rho14, DY480XL, 6-Fam, ferrocene, Fe-complexes, Cu-complexes, Co-complexes, Ni-complexes, etc. In the most preferred embodiment, the short chain was modified by a redox-active label at 3' end of the nucleic acid sequence. The long chain contains a region of hybridization (complementarity) to the short chain and a region of hybridization (complementarity) to the oligonucleotide probe on the electrode surface. In the preferred embodiment, the long chain and the short chains are placed together in equal amounts to produce the oligonucleotide target. The oligonucleotide target melting (dehybridization) temperature is not less than 50°C. In the preferred embodiment for RNAse detection, the oligonucleotide target is composed of a single chain ribonucleic acid molecule (**Fig. 5**). Single chain ribonucleic acid molecule contains the redox-active label as described above. In the most preferred embodiment, the single chain ribonucleic acid molecule was modified by a redox-active label at 3' end of the nucleic acid sequence and contains a region of hybridization (complementarity) to the oligonucleotide probe on the electrode surface.

**A specific design electrode surface.** An electrode is an electroconductive tube or plate in a contact with the solution with one end (part) and in contact with the electrochemical potential-current measuring device with the other part (end). In the preferred embodiment, the electrode is an inert metal (e.g., gold, platinum, etc.) or carbon-based (e.g., graphite, glassy carbon, etc.) cylinder which is isolated from the environment using nonconducting material (e.g., Teflon, polypropylene, etc.) with only the active surface left uninsulated and in contact with the solution. In the most preferred embodiment, the electrode is a screen-printed single-used plate, the surface of which is made from Au metal. The electrode surface is the part of the electrode in a contact with the environment (solution) where the chemical reactions take place. A specific design electrode surface is a surface containing nanomaterials, immobilized oligonucleotide probe and supplemental chemical compounds. In a preferred embodiment, the nanomaterials are used such as metallic nanoparticles (gold, platinum, copper nanoparticles, etc.), carbon nanomaterials (carbon nanotubes, carbon paste, graphene, etc.) electroconductive polymers (polyaniline, polypyrrole, polythiophene, etc.). Nanomaterials increase the electrode surface area and allow for greater immobilization of oligonucleotide probe. In a preferred embodiment, nanomaterials can be placed on the electrode by a drop cast method or can be synthesized directly on the electrode by the means of chemical or electrochemical synthesis. The oligonucleotide probe is a nucleic acid molecule containing 10-200 nucleotides which can hybridize (is complimentary) to the long chain to the oligonucleotide target. The oligonucleotide probe is immobilized on the electrode-nanomaterial surface. In the preferred embodiment, the oligonucleotide probe is immobilized using covalent bond to the electrode surface or/and to the nanomaterial-modified electrode surface. In the most preferred embodiment, the oligonucleotide probe is modified by reduced thiol (HS-) or oxidized thiol (disulfide) (-S-S-) functional group at 5' and/or 3' end of the nucleic acid sequence. In the most preferred embodiment, the modification is HS-alkyl-based (e.g., HS-ethyl, HS-propyl, HS-butyl, HS-heptyl, HS-hexyl etc.) or HS-aromatic (e.g., HS-benzyl, HS-benzyl etc.). The modification of the oligonucleotide probe by a thiol group allows the covalent immobilization of oligonucleotide probe on the gold-based electrode surface or/and gold-based nanomaterial gold surface. The immobilization of oligonucleotide probe on the surface can also be achieved, but not limited to biotin-streptavidin system, carbodiimide-based chemistry via carboxyl, aldehyde, sulfonic, epoxy or isothiocyanate groups, etc. Supplemental chemical compounds are small molecular mass molecules immobilized on the electrode surface after and/or during the oligonucleotide probe immobilization by the means of covalent immobilization and/or physical adsorption. In the preferred embodiment, supplemental chemical compounds are covalently immobilized utilizing the reduced thiol (HS-) functional group to the gold-based electrode surface or/and gold-based nanomaterial gold surface. In the most preferred embodiment, the supplemental chemical compounds belong to the modified-alkanethiol group (e.g. HS-ethyl-modification, HS-propyl-modification, HS-butyl-modification, HS-heptyl-modification, HS-hexyl-modification and etc.) or HS-aromatic-modification (e.g., HS-benzyl-modification, HS-phenyl-modification, HS-naphthyl-modification and etc.), where modification is polar and/or charge containing chemical group such as, but not limited to, hydroxyl, carboxyl, nitro, amino, etc. The supplemental chemical compounds block the unoccupied electrode surface and prevent the events of non-specific and non-selective adsorption (**Fig. 6**).

**Hybridization, and specific signal analysis method.** A hybridization event between the electrode modified with the oligonucleotide probe and the oligonucleotide target is produced and detected by using specific hybridization conditions and specific signal analysis method in a specific sequence. In a preferred embodiment, the hybridization of oligonucleotide probe and oligonucleotide target is carried out by placing electrode together with the sample solution, containing oligonucleotide target. The hybridization event is conducted utilizing the specific buffer solution to increase the rate of the hybridization between the oligonucleotide probe and the oligonucleotide target. In the most preferred embodiment, the hybridization buffer solution is a water-based solution containing high salt concentrations of 0.5-3.5 mol/l, involving but not limited to, sodium chloride, potassium chloride, sodium phosphate, potassium phosphate, TRIS-HCl, calcium chloride. In the most preferred embodiment, the hybridization buffer solution contains 0-1.0 % specific detergents such as, but not limited to, Triton X-100, sodium dodecyl sulfate, IGEPAL CA-630. In the most preferred embodiment, the hybridization buffer solution contains 0-0.1 mol/l of two-valence ions such as Be, Mg, Ca, Sr, Mn, and the temperature used during the hybridization is in range 25-50 °C to increase the rate of hybridization. The hybridization event is detected due to the oligonucleotide target having a redox-active label present in a short chain. The redox-active label becomes immobilized at a very close proximity from the electrode surface and can be detected using methods of electrochemistry, spectrometry, including but not limited to surface plasmon resonance, and/or microscopy. In a preferred embodiment, the redox-active label is detected using the electrochemical methods such as voltammetry, potentiometry, coulometry, impedance and other. In a most preferred embodiment, the redox-active label is detected by using cyclic voltammetry. The potential is cycled in an electrochemical potential range to reduce and oxidize the redox-active label and the reduction and oxidation events can be observed by utilizing electrochemical potential-current measuring device (**Fig. 7**). In the most preferred embodiment, the reductive and oxidative current values correspond to the concentration and/or amount of the redox-active label on the electrode surface. In **Fig. 7** A, B and C parts it is demonstrated how the oxidative and reductive current module values increase depending on the hybridization time. The current value measurement is taken every five minutes. The specific analysis is conducted to estimate the concentration and/or amount of the redox-active label and thus the concentration and/or amount of oligonucleotide target. In the preferred embodiment, the analysis is conducted by estimating the absolute amount of the redox-active label attached to electrode. The absolute amount is estimated by taking the specific range of electrochemical potential-current values from the electrochemical potential-current data points and fitting the received data points to the electrochemical potential-current curve. The fitted curves allow to estimate the amount and/or concentration of the redox-active label and the amount and/or concentration of the oligonucleotide target.

**Kits.** The Invention further includes kits for the detecting and quantifying nuclease-like activity enzyme(s) in a sample. The kit is composed and includes the pre-fabricated single-use screen-printed electrodes containing the specific design surface (as described above), the oligonucleotide target, the nuclease-free water, the nuclease-free plastic tubes and the related measurement and signal analysis algorithm (as described above) which could be applied to standard laboratory potentiostat or any electrochemical potential-current measuring device. Kits could also include a custom-build electrochemical potential-current measuring device together with the factory integrated measurement and signal analysis algorithms.

## Examples

### Example 1: DNAse-like activity detection and quantification in laboratory buffer solutions

Nuclease-free water, Tris(2-carboxyethyl) phosphine hydrochloride (TCEP), 6-Mercapto-1-hexanol and potassium hydroxide (KOH) were purchased from Sigma-Aldrich. Sodium dihydrogen phosphate ($NaH_2PO_4$) and di-sodium hydrogen phosphate ($Na_2HPO_4$) were obtained REACHEN SLOVAKIA. Magnesium chloride hexahydrate ($MgCl_2\,6H_2O$), calcium chloride ($CaCl_2$) and sulfuric acid ($H_2SO_4$) were purchased from Merck. Sodium chloride (NaCl) and TRIS were obtained from ROTH. DNAse I (1000 U/mL, RNase-free) was purchased from ThermoFisher Scientific. Gold nanoparticles (AuNP) were synthesized using procedures as described (Kimling et al., 2006). The solutions used in this research were: i) nuclease working buffer solution (NWBS) (composed of 10 mmol/L TRIS buffer solution with 2.5 mmol/L $MgCl_2$ and 0.5 mmol/L $CaCl_2$, pH - 7.66), hybridization working buffer solution (HWBS) (composed of 20 mmol/L phosphate buffer solution with 3 M NaCl, pH - 7.00), TCEP solution (composed of 20 mM TCEP, 10 mM TRIS, 100 mM $MgCl_2$, pH - 7.2), and immobilization working buffer solution (IWBS) (composed of 10 mM TRIS buffer solution with 100 mM $MgCl_2$, pH - 7.20).

The nucleic acid sequences (oligonucleotides) were purchased from Metabion International AG and used as received without additional purification procedures. The sequences are presented in Table 1.

**Table 1. Nucleic acid sequences**

| Nucleic acid | Sequence |
|---|---|
| Short chain (SEQ ID No. 1) | 5'-CGACTACTGCTCTCGGGC-3'-Atto-MB |
| Long chain (SEQ ID No. 2) | 5'-GCCCGAGAGCAGTAGTCGTTAAGTCTGTTTGTGTTTGATAATT-3' |
| Oligonucleotide probe (SEQ ID No. 3) | (SH-C6)-5'- AATTATCAAACACAAACAGACTTAA -3' |

Oligonucleotide solutions were prepared as described. Freeze-dried oligonucleotides were dissolved in 10 mM TRIS, pH 8.00 solution prepared using nuclease-free water. Oligonucleotide storage concentration was 800 $\mu$mol/L, this solution was kept at -20 °C. Oligonucleotide probe solution was prepared as described. Prior to the immobilization of oligonucleotide probe on the cleaned electrode surface, disulfide bonds of the commercially obtained oligonucleotide were cleaved: probe stock solution was diluted using TCEP solution 1:4 (V:V) so that the concentration of the reducing agent was 100 times higher than that of the oligonucleotides. This mixture was kept at room temperature in the dark for 1 hour and used at once without storing. Oligonucleotide target solution was prepared by mixing a long chain oligonucleotide (24 $\mu$L, 400 $\mu$mol/L) was with a short chain oligonucleotide (25.2 $\mu$L, 400 $\mu$mol/L) along with 10 mmol/L TRIS pH 8.0 buffer solution (190.8 $\mu$L) in a sterile amber glass autosampler tube and heated to 65°C for 10 min with gentle agitation. Afterwards the solution was allowed to cool down to room temperature with gentle agitation over 1 hour. The resulting oligonucleotide target solution (240 $\mu$L) was stored at 4°C.

Electrodes with the immobilized oligonucleotide probe were prepared using the described procedures. At first, a mechanical cleaning was conducted. Gold working electrodes (d=2.0 mm) obtained from PalmSens were rinsed with deionized water and dabbed gently using a lab tissue soaked with acetone. The electrodes were polished on microcloth wetted with deionized water and 0.3 $\mu$m alumina suspension for several minutes and sonicated for 5 minutes in deionized water. Afterwards, the electrochemical cleaning was performed utilizing three electrode electrochemical system with a platinum wire as a counter electrode and Ag/AgCl reference electrode (3.0 mol/L AgCl, E=0.22 V vs. SHE) connected to Gamry Reference™ 600 potentiostat in a 20 mL glass electrochemical cell. The electrodes were placed in 0.05 mol/L KOH solution and 40 cyclic voltammetry cycles were recorded in potential range from 0 to - 2.6 V at 300 mV/s scan rate. Afterwards, the electrodes were rinsed thoroughly and placed in a cell with 0.5 mol/L $H_2SO_4$ solution, and 40 cyclic voltammetry cycles were recorded in potential range from -0.2 to 1.75 V at 300 mV/s scan rate. Then the electrodes were modified by gold nanoparticles. Briefly, 2.0 $\mu$L of gold nanoparticle (AuNP) solution were placed on electrochemically cleaned electrode surface and left to dry at room temperature. Once completely dry, the electrodes were electrochemically cleaned in $H_2SO_4$ as described above. Finally, the electrodes were modified with oligonucleotide probe and supplemental chemical compound (6-mercapto-1-hexanol). Electrodes were immersed in 120 $\mu$L of 1 $\mu$mol/L probe solution (prepared in a 2.0 mL LoBind eppendorf tubes) and kept in the fridge (+4°C) for 12 hours. Afterwards the electrodes were removed from the fridge, rinsed thoroughly using HWBS and submerged in 4.0 mmol/L 6-mercapto-1-hexanol solution (prepared using nuclease-free water) for 60 minutes at room temperature. After another thorough rinse with HWBS, the electrodes were ready for measurements.

Cleavage of oligonucleotide target was carried out by placing the oligonucleotide target with the solutions of DNAse I with various activities. Briefly, a standard DNAse I solution (activity 1000 U/mL) was diluted with the NWBS to give final activities of 0.05 U/mL, 0.01 U/mL, 0.003 U/mL, 0.0003 U/mL, and 0.0001 U/mL with a final volume of 3.46 mL. Afterwards, an oligonucleotide target solution (5 $\mu$l, 40 $\mu$M) was mixed gently with solution of DNAse I (various activities) and incubated with using gentle agitation (300 rpm) at 37°C for 0.5-2 hours (unless stated differently). After the cleavage, the resulting solution was placed to a HWBS (3.5 mL) in a thermostated glass cell and the real-time hybridization measurement was conducted immediately.

Realtime hybridization monitoring of the oligonucleotide target was observed by measuring the oxidation and reduction of the redox-active label (atto methylene blue) immobilized to the 3' end of the short chain of the oligonucleotide target. Oligonucleotide target was placed with the surface-modified electrode utilizing three electrode scheme with a platinum wire as a counter electrode and Ag/AgCl reference electrode (3.0 mol/L AgCl, E=0.22 V vs. SHE). Electrodes were placed into a thermostated glass cell kept at 45°C using the magnetic stirrer (700 rpm) connected to the potentiostat. The potentiostat was programmed to perform a cyclic voltammetry scan (from 0 V to -0.42 V vs. Ag/AgCl, scan rate 50 mV/s, 1mV resolution, 1 cycle) every 5 minutes. The potentiostat was configured to send a digital trigger signal to the magnetic stirrer to turn the stirring off 5 s prior to the cyclic voltammetry scan and again to turn it on 1 second after the cyclic voltammetry scan. The cyclic voltammetry scan was repeated 7 times to a total of 30 minutes. In some experiments, the cyclic voltammetry scan was repeated 11 times to a total of 50 minutes. After the seventh data acquisition event (30-minute mark) the intact oligonucleotide target solution (6 $\mu$l, 40 $\mu$M) was added to a cell as a standard addition procedure to control and evaluate possible sample matrix effects on the electrochemical signal.

The surface concentration of oligonucleotide target was calculated by measuring the reduction peak of the oligonucleotide target. Briefly, the received data points from the cyclic voltammetric measurements were analysed to receive the data points around the reduction process peak. The data points were fitted to a modified electrochemical potential-current curve of the redox-active adsorbed species on the electrode received from (Bard and Faulkner, 2001) (Equation 1). The equation was modified by addition of the third order polynomic function to account for a background current:

$$i = -\frac{n^2 F^2 v\,[Olig]\,Exp\left[\frac{nF}{RT}\,(E-E_0)\right]}{RT\left(1 + Exp\left[\frac{nF}{RT}\,(E-E_0)\right]\right)^2} + a_0 + a_1(E-E_0) + a_2(E-E_0)^2 + a_3(E-E_0)^3$$

(Equation 1)

Here, $I$- current (A), $n$- electron number participating in a reaction (1), $F$- Faraday constant (96485 C/mol), $v$- electrochemical potential scan rate (0.05 V/s), $R$ - gas constant (8.3145 J/(K mol)), $T$- temperature (310 K), $E$ - electrode electrochemical potential, $E_0$- standard redox potentials of atto methylene blue (around -0.27 V vs. Ag/AgCl), $a_0$-$a_3$ - background current function coefficients, $Olig$ - oligonucleotide target amount on the electrode surface (mol). The fitting allows to calculate the value of $Olig$ and estimate the hybridization rate of oligonucleotide target.

Samples were prepared to measure the nuclease-like activity in laboratory water (nuclease-free). For the nuclease-like activity, a model enzyme - nonspecific nuclease DNAse I was used. Samples were prepared with different activity of DNAse I: 0.0001 U/mL, 0.0003 U/mL, 0.003 U/mL, 0.01 U/mL, 0.05 U/mL. The oligonucleotide target was placed with the samples and kept for 30 min at 37°C. The received solutions were placed to a cell with the prepared electrode and allowed to hybridize for 30 min. The hybridization was monitored by measuring the concentration of the oligonucleotide target on the electrode surface. The summary of the results is presented in **Fig. 8.** The figure demonstrates that the relative signal of the sensor, i.e., relative hybridization rate of the oligonucleotide target, is directly related to the nuclease-like activity of the solution. In case high activity of DNAse I was present (e.g., 0.05 U/mL), the hybridization rate was almost equal to zero, since the enzyme cleaved all the oligonucleotide target present in a solution. The decrease in DNAse I activity resulted in the increase in the signal of the sensor up to 0.0001 U/mL. The native logarithm of nuclease activity was linearly correlated with the electrode signal ($R^2$=0.98) and allowed to produce a calibration curve. According to the calibration curve and zero values of the sensor, the limit of the detection of the electrode, for this example, was calculated 0.000036 U/mL. To summarize, the electrode was applied to measure nuclease-like activity in laboratory water in nuclease-like activity range 0.000035-0.05 U/mL calibrated against the activity of DNAse I.

**Example 2: DNAse-like activity detection in quantification in human saliva samples**

DNase-like activity detection and quantification in human saliva samples was conducted using the procedures and materials as described in Example 1. Additionally, only the saliva samples were prepared as described. Saliva samples were collected in the morning into a sterile polypropylene tube from a volunteer donor. The saliva donor did not have any foods or drinks for at least one hour prior to the collection of samples. A saliva sample (800 $\mu$L) was centrifuged at 1600 rcf for 10 minutes and again at 16 000 rcf for 10 minutes. The received saliva supernatant (200 $\mu$L) was placed into NWBS (3.3 mL) along with oligonucleotide target solution (4 $\mu$L, 40 $\mu$mol/L) in a test tube and mixed gently by inverting 40 times. The resulting solution was incubated with 300 rpm agitation at 37 °C for 2 hours. The cleaved solution was mixed with HWBS (3.5 mL) in a thermostated glass cell, and the electrode response was measured immediately.

Saliva sample was placed with the oligonucleotide target and kept for 2 h at 37 °C. The received solution was placed to a cell with the prepared electrode and allow to hybridize for 30 min. The hybridization was monitored by measuring the concentration of the oligonucleotide target on the electrode surface. The summary of the results is presented in **Fig. 9** and compared to the results received when standard activities of nuclease DNAse I was used. **Fig. 9** demonstrates that saliva sample has a nuclease-like activity which is higher compared to activity of 0.01 U/mL DNAse I solution, but significantly lower compared to activity of 0.0001 U/mL DNAse I. From the calibration curve and the dilution of the sample (17.5 dilution ratio) the estimated nuclease-like activity of the saliva sample was 0.19 U/mL. To conclude, the electrode was applied to measure nuclease-like activity in human saliva sample.

**Example 3: DNAse-like activity detection in human blood samples**

DNase-like activity detection in human blood samples was conducted using the procedures and materials as described in Example 1. Additionally, only the blood samples were prepared as described. Blood sample was received from a volunteer donor in a morning. A blood sample (300 $\mu$L) was placed together with oligonucleotide target (5 $\mu$L) in NWBS (3.2 ml), the concentration of oligonucleotide target was 57 nmol/L. Afterwards, the solution was kept for 1 h at 37 °C with gentle agitation (300 rpm). The received solution was placed to a cell with the prepared electrode in a HWBS with 0.2 mol/L $Na_2SO_3$ and measured immediately.

Blood sample was placed with the oligonucleotide target and kept for 1 h at 37 °C. The hybridization of the oligonucleotide target on the electrode surface was monitored for 30 min. The summary of the results is presented in **Fig. 10** and compared to the results received when standard activities of nuclease DNAse I was used. **Fig. 10** demonstrates that blood sample has a nuclease-like activity which is much higher compared to activity of 0.01 U/mL DNAse I solution, but lower compared to activity of 0.0001 U/mL DNAse I. From the calibration curve and the dilution of the sample (11.7 dilution ratio) it was estimated that nuclease-like activity in blood sample can be detected.

**Example 4: DNAse detection and quantification on surfaces and surface contamination measurements**

DNase-like activity detection and quantification on surface samples was conducted using the procedures and materials as described in Example 1. Additional procedures were applied as described. A sterile synthetic wipe containing 70% isopropanol-water was purchased from a store and a small diagonal cut was made in the package under the sterile conditions. The package was placed flat in a clean fume hood to minimize contact with air and the solvent was allowed to evaporate. Afterwards, the wipes were stored in sterile zip-lock bags. Prior to the collection of surface samples the wipes were cut to size ($5.25mm^2$) under sterile conditions and moistened with NWBS (300 $\mu$L) inside a sterile zip-lock bag, sealed and transported to the surface to investigate. The wipe was handled with sterile nitrile gloves and a representative surface (450 $cm^2$) was gently swabbed with both sides using diagonal side-to side motion, after which the wipe was re-sealed in the zip-lock bag and transported to the laboratory. The wipes were placed into a test tube and extracted with two portions of NWBS (1 mL*2) by pipette mixing and inverting for 1 minute each. The extracted sample fractions were collected into test tubes and placed with the oligonucleotide target (9 $\mu$L, 8 $\mu$mol/L total). The resulting test tubes were mixed by inverting 40 times. The resulting solution was incubated with 300rpm agitation at 37 °C for 4 hours. The received solution was mixed with HWBS (2 ml) in a thermostated glass cell and measured immediately.

Samples of the investigated surfaces were placed together with the oligonucleotide target, kept for 4 h at 37 °C. The hybridization of the oligonucleotide target on the electrode surface was monitored for 30 min. The summary of the results is presented in **Fig. 11** and compared to the results received when standard activities of nuclease DNAse I was used. At first, the sterile surface was analysed. The sterile surface was a surface which was thoroughly cleaned with isopropyl alcohol to ensure no nuclease-like activity contamination should be present. The **Fig. 11** demonstrates that the sterile surface had no detectable nuclease activity, and the relative signal was similar to the control. Afterwards the different surfaces were analysed: Surface 1 - a touchscreen surface of a printer, Surface 2 - a table surface in a resting room, Surface 3 - surface of a computer keyboard, Surface 3 - surface of another computer keyboard. The analysis demonstrated that all of the analysed surfaces were contaminated with the nuclease-like activity exhibiting enzymes (with the exception of a sterile surface). To add more, the correlation appeared between the nuclease-like activity and the surface cleanness. For example, the surface with highest nuclease-like activity (Surface 2, activity > 0.05 U/mL) was detected on the table in a commonly used room. This result could be expected since this surface is used for food preparation, eating and other common activities. The nuclease-like activity for different surfaces was estimated: Sterile surface <0.000063 (lower than LOD), Surface 1 - 0.011 U/mL, Surface 2 - >0.05 U/mL, Surface 3 - 0.0019 U/mL, Surface 4 - 0.0013 U/mL.

**Example 5: RNAse-like activity detection and quantification in laboratory buffer solutions**

RNase-like activity detection and quantification in laboratory buffer solutions was conducted using the procedures and materials as described in Example 1. The solutions used for ribonuclease activity include: ribonuclease working buffer solution (20 mM PBS, 20 mM NaCl, pH 6), RNAse stock solution (1000 KU/ml RNAse A, 50 mM Tris, 50% glycerol, pH - 7.00), RNA nucleotide stock solution (40 $\mu$M Oligonucleotide target, 20 mM Tris, pH 7.00). Lyophilized ribonuclease A from bovine pancreas and glycerol were purchased from Sigma-Aldrich. RNA nucleic acid sequence (oligonucleotide target) was purchased from Metabion International AG and used as received without additional purification procedures. The sequence is presented in Table 1.

**Table 1. Nucleic acid sequences**

| Nucleic acid | Sequence |
|---|---|
| Oligonucleotide target (SEQ ID No. 4) | (Atto-MB)-5'-ggcagggcagggcagucguuaagucuguuuguguuugauaauu-3' |

Samples were prepared to measure the nuclease-like activity in laboratory water (nuclease-free). For the nuclease-like activity, a model enzyme - nonspecific nuclease RNAse A was used. Samples were prepared with different activity of RNAse A: $10^{-7}$ KU/mL, $10^{-6}$ KU/mL, $10^{-5}$ KU/mL. The oligonucleotide target was placed with the samples and kept for 30 min at 37°C. The received solutions were placed to a cell with the prepared electrode and allowed to hybridize for 30 min. The hybridization was monitored by measuring the concentration of the oligonucleotide target on the electrode surface. Results demonstrated, that the relative signal of the sensor, i.e., relative hybridization rate of the oligonucleotide target, is directly related to the nuclease-like activity of the solution. In case high activity of RNAse A was present (e.g., $10^{-5}$ KU/mL), the hybridization rate was almost equal to zero, since the enzyme cleaved all the oligonucleotide target present in a solution. The decrease in RNAse A activity resulted in the increase in the signal of the sensor up to $10^{-7}$ KU/mL. To summarize, the electrode was applied to measure nuclease-like activity in laboratory water in nuclease-like activity range $10^{-7}$ KU/mL - $10^{-5}$ KU/mL calibrated against the activity of RNAse A.

**Example 6: Detection and quantification of DNAse-like activity in commercial protein matrixes**

DNase-like activity detection and quantification in commercial protein matrixes was conducted using the procedures and materials as described in Example 1 with additional procedures as described. Three commercial proteins matrixes were purchased: glucose oxidase (EC 1.1.3.4 from *Aspergillus niger,* from Sigma), papain (from Merck) and bovine serum albumin (BSA) (from Fluka). For the determination of nuclease-like activity in commercial protein matrixes, protein crystals were diluted in NWBS to a final concentration of 1 mg/mL. Solutions of samples were taken (3.6 mL) and to these solutions oligonucleotide target was added (5 $\mu$L, 40 $\mu$mol/L) to a final concentration of 55.6 nmol/L. Afterwards, the mixtures were gently agitated at 300 rpm at 37 °C for 30 minutes. The resulting solution was then diluted 1:1 (V:V) using HWBS and electrochemical measurements were performed. The final concentration in the measurement cell was thus 0.5 mg/mL of the assayed protein.

Samples of the investigated commercial protein matrixes were placed with the oligonucleotide target and kept for 0.5 h at 37°C. The received solutions were placed to a cell with the prepared electrode and allow to hybridize for 30 min. The hybridization was monitored by measuring the concentration of the oligonucleotide target on the electrode surface. The summary of the results is presented in **Figure 12** and compared to the results received when standard activities of nuclease DNAse I was used. The different protein matrixes gave different results. The relative signal of electrode was similar to the control only in case the BSA protein matrix was used (around 1.0), thus no DNAse-like activity was detected in the investigated protein matrix (lower than LOD, <0.000063 U/mL calibrated against DNAse I). The significant contamination of nuclease-like activity enzymes was detected as well as quantified in both protein matrixes of glucose oxidase and papain. In case of glucose oxidase, the nuclease-like contamination was significant - the signal value was around 0, thus it indicated the nuclease-like activity higher than 0.05 U/mL (calibrated against DNAse I). In case or papain sample, the electrode relative signal was around 0.33 and was related to the nuclease-like activity of 0.0058 U/mL (calibrated against DNAse I). To conclude, the electrode was applied to measure (i.e., detect and quantify) nuclease-like activity of commercial protein matrixes.

**References**

1. Bard, A.J., Faulkner, L.R., 2001. Electrochemical methods: fundamentals and applications, 2nd ed. ed. Wiley, New York.

2. Ding, J., Qin, W., 2013. Potentiometric sensing of nuclease activities and oxidative damage of single-stranded DNA using a polycation-sensitive membrane electrode. Biosensors and Bioelectronics 47, 559-565. https://doi.org/10.1016/j.bios.2013.03.066

3. Flenker, K.S., Burghardt, E.L., Dutta, N., Burns, W.J., Grover, J.M., Kenkel, E.J., Weaver, T.M., Mills, J., Kim, H., Huang, L., Owczarzy, R., Musselman, C.A., Behlke, M.A., Ford, B., McNamara, J.O., 2017. Rapid Detection of Urinary Tract Infections via Bacterial Nuclease Activity. Molecular Therapy 25, 1353-1362. https://doi.org/10.1016/j.ymthe.2017.03.015

4. He, Y., Xiong, L.-H., Xing, X.-J., Tang, H.-W., Pang, D.-W., 2013. An ultra-high sensitive platform for fluorescence detection of micrococcal nuclease based on grapheneoxide. Biosensors and Bioelectronics 42, 467-473. https://doi.org/10.1016/j.bios.2012.10.045

5. Kimling, J., Maier, M., Okenve, B., Kotaidis, V., Ballot, H., Plech, A., 2006. Turkevich Method for Gold Nanoparticle Synthesis Revisited. The Journal of Physical Chemistry B 110, 15700-15707. https://doi.org/10.1021/jp061667w

6. Kruspe, S., Dickey, D.D., Urak, K.T., Blanco, G.N., Miller, M.J., Clark, K.C., Burghardt, E., Gutierrez, W.R., Phadke, S.D., Kamboj, S., Ginader, T., Smith, B.J., Grimm, S.K., Schappet, J., Ozer, H., Thomas, A., McNamara, J.O., Chan, C.H., Giangrande, P.H., 2017. Rapid and Sensitive Detection of Breast Cancer Cells in Patient Blood with Nuclease-Activated Probe Technology. Molecular Therapy - Nucleic Acids 8, 542-557. https://doi.org/10.1016/j.omtn.2017.08.004

7. Sato, S., Fujita, K., Kanazawa, M., Mukumoto, K., Ohtsuka, K., Takenaka, S., 2009. Reliable ferrocenyloligonucleotide-immobilized electrodes and their application to electrochemical DNase I assay. Analytica Chimica Acta 645, 30-35. https://doi.org/10.1016/j.aca.2009.04.047

8. Sato, S., Takenaka, S., 2014. Highly Sensitive Nuclease Assays Based on Chemically Modified DNA or RNA. Sensors 14, 12437-12450. https://doi.org/10.3390/s140712437

9. Senavirathne, G., Liu, J., Lopez, M.A., Hanne, J., Martin-Lopez, J., Lee, J.-B., Yoder, K.E., Fishel, R., 2015. Widespread nuclease contamination in commonly used oxygen-scavenging systems. Nat Methods 12, 901-902. https://doi.org/10.1038/nmeth.3588

10. Zhang, H., Cheng, C., Dong, N., Ji, X., Hu, J., 2021. Positively charged Ag@Au core-shell nanoparticles as highly stable and enhanced fluorescence quenching platform for one-step nuclease activity detection. Biochemical Engineering Journal 167, 107890. https://doi.org/10.1016/j.bej.2020.107890

11. Patent no: EP2751567B1

12. Patent no: US7276337B2

13. Patent no: US2020/0017898A1

14. Patent no: WO2014/207515A1

**Claims**

1. A system for nuclease-like enzymatic activity detection comprising:

   (i) an electrode surface together with an immobilized oligonucleotide probe and supplemental chemical compound(s), and
   (ii) a medium for an oligonucleotide target.

2. The system according to claim 1, wherein an oligonucleotide target is a nucleic acid molecule of DNA or RNA origin, wherein in a case of DNA, the oligonucleotide is composed of single-stranded and double-stranded nucleic acid chains of two nucleic acid sequences of different length - the short chain composed of 10-100 nucleotides containing a complementary region to the long chain and the long chain, composed of 10-200 nucleotides containing a complementary region to the short chain and a complementary region to an oligonucleotide probe, wherein in a case of RNA - oligonucleotide is composed of single-stranded sequence with a complementary region to an oligonucleotide probe.

3. The oligonucleotide target according to claims 1-2, wherein it comprises at least one cleavage site for nuclease-like activity enzyme(s), and wherein it is kept with a sample containing enzymes with nuclease-like activity for 0.5-12 h at 30-40 °C, in a water-based buffer solution containing low salt concentration of 0-0.05 mol/L with the two-valence ions such as Be, Mg, Ca, Sr, Mn at concentration of 0-0.05 mol/L.

4. The oligonucleotide target according to claims 1-3, wherein it contains a redox-active label at the end of 3' and/or 5' or in a middle of the chain.

5. The oligonucleotide target according to claims 1-4, where the redox-active label is a molecule capable of a reversible electron and/or proton transfer and existing in at least two stable forms and comprising redox-active molecules such as methylene blue, AttoMB2, IRDye®800, IRDye®700, Yakima Yellow®, Texas Red®, Atto Rho13, Cy5.5, Cy5-azide, Azide-fluor 488, Atto655, Atto647N, Atto Rho14, DY480XL, 6-Fam, ferrocene, Fe-complexes, Cu-complexes, Co-complexes, or Ni-complexes.

6. The oligonucleotide target according to claims 1-5, which has the dehybridization temperature of no less than 50°C.

7. The system according to claim 1, wherein the electrode is an electroconductive cylinder or a plate in contact with the electrochemical potential-current measuring device with one side and in a contact with the solution with the other side - the surface, which is composed of metals such as gold, platinum, or carbon-based materials such as graphite, or glassy carbon.

8. The electrode according to claim 7, wherein the surface nanomaterials are selected from metallic nanoparticles such as gold, platinum, and copper, or carbon nanomaterials such as carbon nanotubes, carbon paste, and graphene, or electroconductive polymers such as polyaniline, polypyrrole, and polythiophene.

9. The electrode according to claims 7-8, which has a surface involving an immobilized oligonucleotide probe, which is a nucleic acid molecule composed of 10-200 nucleotides containing reduced thiol (HS-) and/or oxidized thiol (-S-S-) functional group at 5' and/or 3' end of the nucleic acid sequence and a complementary region to the oligonucleotide target.

10. The oligonucleotide probe according to claims 1 and 9, wherein the reduced thiol (HS-) and/or oxidized thiol (-S-S-) functional group is a HS-alkyl-based molecule such as HS-ethyl, HS-propyl, HS-butyl, HS-heptyl, or HS-hexyl, and/or an HS-aromatic such as HS-benzyl, HS-phenyl, or HS-naphthyl.

11. The oligonucleotide probe according to claims 1, 9-10, which can be immobilized to the electrode surface via thiol, biotin-streptavidin, carbodiimide-based, carboxyl, aldehyde, sulfonic, epoxy, or isothiocyanate group-based chemistry.

12. The electrode surface according to claims 1, 9-10, wherein the supplemental chemical compound is a small molecular mass compound selected from the group of modified-alkanethiols with modifications such as HS-ethyl-modification, HS-propyl-modification, HS-butyl-modification, HS-heptyl-modification, HS-hexyl-modification, or HS-aromatic-modification such as HS-benzyl-modification, HS-phenyl-modification, HS-naphthyl-modification, where the modification is nonpolar, polar and/or charge containing chemical group such as, but not limited to, hydroxyl, carboxyl, nitro, or amino.

13. The system according to claims 1-12, wherein enzymatic activity is detected by measuring the concentration and/or amount of the redox-active label described in claim 5 by the means of electrochemical detection such as voltammetry, potentiometry, coulometry or impedance measuring the current dependence of electrochemical potential, current dependence on time, electrochemical potential dependence on time, charge dependence on time, charge dependence on electrochemical potential, impedance dependence on time, impedance dependence on electrochemical potential, impedance dependence on frequency from the instrumental data points.

14. The system according to claims 1-13, which allows to detect and quantify, using the received calibration curve, the nuclease-like activity exhibiting enzymes in various media, involving but not limited to, laboratory-based buffer solutions, human saliva, human blood, samples taken from laboratory surfaces, samples taken from commercial enzyme mixes (matrixes).

## Oligonucleotide target (DNAse detection)

Nuclease-like
enzymatic activity (DNAses)

Oligonucleotide target (cleaved)

## Oligonucleotide target (RNAse detection)

Nuclease-like
enzymatic activity (RNAses)

Fig. 1

Fig. 2

Fig. 3

Fig. 4

Short chain

Long chain

Oligonucleotide target

Oligonucleotide probe

Fig. 5

Fig. 6

Fig. 7

Fig. 8

Fig. 9

**Fig. 10**

**Fig. 11**

Fig. 12

**Europäisches Patentamt**
**European Patent Office**
**Office européen des brevets**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | SATO S ET AL: "Reliable ferrocenyloligonucleotide-immobilized electrodes and their application to electrochemical DNase I assay", ANALYTICA CHIMICA ACTA, ELSEVIER, AMSTERDAM, NL, vol. 645, no. 1-2, 10 July 2009 (2009-07-10), pages 30-35, XP026143639, ISSN: 0003-2670, DOI: 10.1016/J.ACA.2009.04.047 [retrieved on 2009-05-06] * the whole document * * abstract, Fig. 1, 2 * | 1-14 | INV. C12Q1/6825 |
| X,D | SHINOBU SATO ET AL: "Highly Sensitive Nuclease Assays Based on Chemically Modified DNA or RNA", SENSORS, vol. 14, no. 7, 11 July 2014 (2014-07-11), pages 12437-12450, XP055319364, DOI: 10.3390/s140712437 * the whole document * * abstract, Fig. 3c, 4, 8b * | 1-14 | |
| X | US 2002/055103 A1 (BARTON JACQUELINE K [US] ET AL) 9 May 2002 (2002-05-09) * the whole document * * para. 21, 23-24, Fig.1 * | 1-14 | TECHNICAL FIELDS SEARCHED (IPC)  C12Q |
| X | KR 2018 0134651 A (UNIV SOONCHUNHYANG IND ACAD COOP FOUND [KR]) 19 December 2018 (2018-12-19) * the whole document * * para. 1, 14, 27, 33, Fig. 1 * | 1-14 | |

-/--

The present search report has been drawn up for all claims

| Place of search | Date of completion of the search | Examiner |
|---|---|---|
| Munich | 20 June 2022 | Sauer, Tincuta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 15 0566

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/009944 A1 (BOWATER RICHARD [GB] ET AL) 11 January 2007 (2007-01-11) * the whole document * * para. 10, 22, 34, 99, claims 1-8, 13, 14; Fig. 17 * ----- | 1-14 | |

TECHNICAL FIELDS
SEARCHED        (IPC)

The present search report has been drawn up for all claims

| Place of search | Date of completion of the search | Examiner |
|---|---|---|
| Munich | 20 June 2022 | Sauer, Tincuta |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

............................................................................
& : member of the same patent family, corresponding
   document

EPO FORM 1503 03.82 (P04C01)

page 2 of 2

## ANNEX TO THE EUROPEAN SEARCH REPORT
## ON EUROPEAN PATENT APPLICATION NO.

EP 22 15 0566

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2022

| Patent document cited in search report | | Publication date | Patent family member(s) | | Publication date |
|---|---|---|---|---|---|
| US 2002055103 | A1 | 09-05-2002 | AU | 3550699 A | 25-10-1999 |
| | | | DE | 69932236 T2 | 31-05-2007 |
| | | | EP | 1068359 A1 | 17-01-2001 |
| | | | JP | 2002510791 A | 09-04-2002 |
| | | | US | 6221586 B1 | 24-04-2001 |
| | | | US | 2002055103 A1 | 09-05-2002 |
| | | | WO | 9951778 A1 | 14-10-1999 |
| KR 20180134651 | A | 19-12-2018 | NONE | | |
| US 2007009944 | A1 | 11-01-2007 | NONE | | |

EPO FORM P0459

For more details about this annex : see Official Journal of the European Patent Office, No. 12/82

## REFERENCES CITED IN THE DESCRIPTION

### Patent documents cited in the description

- EP 2751567 B1
- US 7276337 B2
- US 20200017898 A1
- WO 2014207515 A1

### Non-patent literature cited in the description

- **BARD, A.J. ; FAULKNER, L.R.** Electrochemical methods: fundamentals and applications. Wiley, 2001
- **DING, J. ; QIN, W.** Potentiometric sensing of nuclease activities and oxidative damage of single-stranded DNA using a polycation-sensitive membrane electrode. *Biosensors and Bioelectronics,* 2013, vol. 47, 559-565, https://doi.org/10.1016/j.bios.2013.03.066
- **FLENKER, K.S. ; BURGHARDT, E.L. ; DUTTA, N. ; BURNS, W.J. ; GROVER, J.M. ; KENKEL, E.J. ; WEAVER, T.M. ; MILLS, J. ; KIM, H. ; HUANG, L.** Rapid Detection of Urinary Tract Infections via Bacterial Nuclease Activity. *Molecular Therapy,* 2017, vol. 25, 1353-1362, https://doi.org/10.1016/j.ymthe.2017.03.015
- **HE, Y. ; XIONG, L.-H. ; XING, X.-J. ; TANG, H.-W. ; PANG, D.-W.** An ultra-high sensitive platform for fluorescence detection of micrococcal nuclease based on grapheneoxide. *Biosensors and Bioelectronics,* 2013, vol. 42, 467-473, https://doi.org/10.1016/j.bios.2012.10.045
- **KIMLING, J. ; MAIER, M. ; OKENVE, B. ; KOTAIDIS, V. ; BALLOT, H. ; PLECH, A.** Turkevich Method for Gold Nanoparticle Synthesis Revisited. *The Journal of Physical Chemistry B,* 2006, vol. 110, 15700-15707, https://doi.org/10.1021/jp061667w
- **KRUSPE, S. ; DICKEY, D.D. ; URAK, K.T. ; BLANCO, G.N. ; MILLER, M.J. ; CLARK, K.C. ; BURGHARDT, E. ; GUTIERREZ, W.R. ; PHADKE, S.D. ; KAMBOJ, S.** Rapid and Sensitive Detection of Breast Cancer Cells in Patient Blood with Nuclease-Activated Probe Technology. *Molecular Therapy - Nucleic Acids,* 2017, vol. 8, 542-557, https://doi.org/10.1016/j.omtn.2017.08.004
- **SATO, S. ; FUJITA, K. ; KANAZAWA, M. ; MUKUMOTO, K. ; OHTSUKA, K. ; TAKENAKA, S.** Reliable ferrocenyloligonucleotide-immobilized electrodes and their application to electrochemical DNase I assay. *Analytica Chimica Acta,* 2009, vol. 645, 30-35, https://doi.org/10.1016/j.aca.2009.04.047
- **SATO, S. ; TAKENAKA, S.** Sensitive Nuclease Assays Based on Chemically Modified DNA or RNA. *Sensors,* 2014, vol. 14, 12437-12450, https://doi.org/10.3390/s140712437
- **SENAVIRATHNE, G. ; LIU, J. ; LOPEZ, M.A. ; HANNE, J. ; MARTIN-LOPEZ, J. ; LEE, J.-B. ; YODER, K.E. ; FISHEL, R.** Widespread nuclease contamination in commonly used oxygen-scavenging systems. *Nat Methods,* 2015, vol. 12, 901-902, https://doi.org/10.1038/nmeth.3588
- **ZHANG, H. ; CHENG, C. ; DONG, N. ; JI, X. ; HU, J.** Positively charged Ag@Au core-shell nanoparticles as highly stable and enhanced fluorescence quenching platform for one-step nuclease activity detection. *Biochemical Engineering Journal,* 2021, vol. 167, 107890, https://doi.org/10.1016/j.bej.2020.107890